# EUROPÄISCHE PATENTSCHRIFT

(11) **EP 0 898 170 B1**
(45) Veröffentlichungstag und Bekanntmachung des Hinweises auf die Patenterteilung: **09.10.2002**
(21) Anmeldenummer: 98113606.2
(22) Anmeldetag: 21.07.1998
(51) Int. Cl.: G01N 33/553, G01N 33/532, G01N 33/58

(54) **Detergenzhaltige Goldkonjugate**
Detergent-containing gold conjugates
Conjugués d'or contenant des tensioactifs

(30) Priorität: 22.07.1997 DE 19731469
(43) Veröffentlichungstag der Anmeldung: 24.02.1999
(73) Patentinhaber: Roche Diagnostics GmbH, 68305 Mannheim-Waldhof (DE)
(72) Erfinder: Nichtl, Alfons Dr., 82383 Hohenpeissenberg (DE)
(74) Vertreter: Huber, Bernhard, Dipl.-Chem.

(56) Entgegenhaltungen:
- EP-A- 0 311 094
- EP-A- 0 489 465
- WO-A-90/13637
- US-A- 5 141 850
- BEHNKE ET AL.: "Non-specific binding of protein-stabilized gold sols as a source of error in immunocytochemistry" EUR. J. CELL. BIOL., Bd. 41, Nr. 2, 1986, Seiten 326-328, XP000881161

## Beschreibung

Die Erfindung betrifft ein Verfahren zur Herstellung von Zusammensetzungen, welche kolloidale Partikel, an deren Oberfläche Biomoleküle adsorbiert sind, und ein Detergenz umfassen.

Konjugate aus Biomolekülen, wie etwa Proteinen oder Nukleinsäuren, und kolloidalen Partikeln werden weit verbreitet beispielsweise als Signalgeber oder/und als Fangreagenz in diagnostischen und therapeutischen Verfahren eingesetzt. Sie dienen beispielsweise als Marker in Nachweisverfahren, wie etwa Immunoassays oder als Mikroprojektile zum Gentransfer. Als Partikel können Partikel aus Metallen und Metallverbindungen wie etwa Metalloxiden, Metallhydroxiden, Metallsalzen und Polymerkernen, die mit Metallen oder Metallverbindungen beschichtet sind, verwendet werden (vgl. z.B. US-A-4,313,734; Leuvering et al., J.Immunoassay 1 (1980), 77-91; Leuvering Dissertation (1984), Sol Particle Immunoassay (SPIA) : The Use of Antibody Coated Particles as Labelled Antibodies in Various Types of Immunoassay; Uda et al., Anal.Biochem. 218 (1994), 259-264, DE-OS 41 32 133, Seite 3, Zeilen 16-18, für Anwendungen als Marker und Tang et al., Nature 356 (1992), 152-154; Eisenbraun et al., DNA and Cell Biology 12 (1993), 791-797; Williams et al., Proc.Natl.Acad.Sci. USA 88 (1991), 2726-2730 für Anwendungen zum Gentransfer). Weiterhin ist auch die Verwendung von nichtmetallischen kolloidalen Partikeln wie etwa Carbonteilchen bekannt (van Amerongen, Anabiotic '92 (1993), 193-199). Am häufigsten werden zur Zeit kolloidale Goldpartikel eingesetzt.

Zur Herstellung von Biomolekül-Gold-Konjugaten werden zunächst nach allgemein bekannten Verfahren Goldsole durch Reduktion von Tetrachlorgoldsäure hergestellt. Anschließend erfolgt eine Beladung der Goldsole mit dem jeweils gewünschten Biomolekül, z. B. Proteinen wie Antikörpern, Protein A, Protein G, Streptavidin, etc. Die jeweiligen Beladebedingungen (pH, Puffer, Konzentration der Biomoleküle etc.) richten sich nach dem isoelektrischen Punkt des Biomoleküls, der MPA (minimal protecting amount) oder/und der spezifischen Anwendung des Konjugats (vgl. z. B. De Mey, The Preparation and Use of Gold Probes, in: Immunocytochemistry, Hrsg.: J. M. Polak und S. v. Noorden, Seiten 115-145, Wright, Bristol 1986; J. E. Beesley, Colloidal Gold: A New Perspective for Cytochemical Marking, Microscopy Handbooks 17, Oxford University Press, 1989, insbesondere Seiten 1-14; G. Frens, Nature Physical Science, 241 (1973) 20-22; J. Roth, "The Colloidal Gold Marker System for Light and Electron Microscopic Cytochemistry" in: Immunocytochemistry 2 (1983) 218-284). Auf die Offenbarung dieser Dokumente wird ausdrücklich Bezug genommen.

Nach Beladung der kolloidalen Partikel mit dem jeweils gewünschten Biomolekül ist eine Stabilisierung der Konjugate notwendig. Durch diese Stabilisierung soll eine Aggregation der Partikel minimiert werden und verbleibende freie, der Adsorption zugängliche Oberflächen, abgesättigt werden. Im Stand der Technik werden als Stabilisatoren inerte Proteine, z. B. Rinderserumalbumin, Blutersatzmischungen etc., wasserlösliche technische Polymere wie Polyethylenglykol (Molekulargewicht 20.000 D), Polyvinylpyrrolidon, Polyvinylalkohol, Polyvinylsulfat, Dextran und Gelatine verwendet (vgl. z.B. De Mey, Supra; Beesley, Supra; Behnke, Eur. J. Cell Biol. 41 (1986), 326-338; DE 24 20 531 C3; und Meisel et al., J. Phys. Chem. 85 (1981), 179-187).

EP 0 489 465 A2 beschreibt Goldsolpartikel, die mit einer Alkanthiolverbindung beschichtet sind. Diese Alkanthiolbeschichtung dient zur Bindevermittlung an Biomoleküle, wie etwa Proteine, Antikörper, Antigene etc. Die Herstellung der Konjugate erfolgt dadurch, dass zunächst ein Goldsol mit einem Alkanthiol beschichtet wird. Dabei kann der Alkanthiolbeschichtungslösung ein Detergenz zugegeben werden. Das mit Alkanthiol beschichtete Goldsol wird dann in einem weiteren Schritt mit Biomolekülen beladen. Diese Beladungslösung kann zwar Kopplungschemikalien zur kovalenten Bindung der Biomoleküle an die Thiolbeschichtung umfassen (beispielsweise EDC oder SNHS), es findet sich aber keinerlei Hinweis darauf, dass der Biomoleküle enthaltenden Lösung auch ein Detergenz zugesetzt werden könnte.

US 5,141,850 beschreibt einen Immunoassay unter Verwendung eines an kolloidales Gold konjugierten Antikörpers als Markierung. Die Zugabe eines Detergenz zu einer Biomoleküle enthaltenden Lösung vor der Beladung von kolloidalen Partikeln wird nicht beschrieben. Ein Detergenz kann den fertigen Goldsol-Antikörper-Konjugaten nachträglich zugesetzt werden.

WO 90/13637 betrifft ein lyophilisiertes Gemisch zur Verwendung in einem Immunoassay. Das Gemisch umfasst eine immunoreaktive Komponente, beispielsweise ein Antikörper-Goldsol-Konjugat, eine organische Komponente zur Verbesserung des Immunoassays sowie einen Zucker, der die Agglomeration der organischen Komponente beim Lyophilisieren verhindern soll. Die organische Komponente kann ein Detergenz umfassen. Es wird nicht das Vorliegen von Detergenz bereits vor der Herstellung der Kolloidkonjugate in der Biomolekül-Lösung beschrieben, vielmehr kann ein Detergenz den fertigen Konjugaten nachträglich zugesetzt werden.

EP 0 311 094 A2 beschreibt ein diagnostisches Mittel zur Bestimmung von ApoB, wobei eine Partikeldispersion eingesetzt wird. Die bereits mit Antikörper beladenen Partikel können mit Zusätzen, wie etwa Detergenzien versetzt werden, um eine spontane Agglutination zu vermeiden.

O. Behnke et al., Eur. J. Cell. Biol. 41 (1986), 326-328 beschreibt Untersuchungen hinsichtlich der unspezifischen Bindung von unterschiedlich stabilisierten Goldsolen. Die Herstellung der Goldsole wird auf Seite 327, linke Spalte beschrieben. Dabei wird bei der Konjugation insbesondere auf den pH-Wert geachtet. Nach Adsorption des Proteins an die Goldsolpartikel werden dann verschiedene Stabilisierungsmittel zugegeben, darunter auch Tween 20.

Weiterhin wurde auch die Möglichkeit der Stabilisierung von Goldsolen durch Phosphan-Komplexliganden beschrieben (Schmid et al., Z. Naturforsch. 45b (1994), 989-994).

Bei der Beladung von kolloidalen Goldpartikeln geht man gewöhnlich so vor, dass die Lösung des an das Gold zu adsorbierenden Proteins ebenso wie das Goldsol auf einen pH-Wert nahe des isoelektrischen Punktes (IP) des Proteins eingestellt wird. Als wesentlich für ein erfolgreiches Beladen wird dabei im Stand der Technik erachtet, dass die Proteinlösung möglichst keine Zusätze enthalten darf und z.B. die Ionenstarke nicht über 10 mM liegen darf. Zur Beladung wird die Proteinlösung unter Rühren zum Goldsol zugegeben oder umgekehrt. Nachdem das Protein an die Goldpartikel gebunden ist, wird eine Lösung eines geeigneten Stabilisierungsmittels zugegeben. Gegebenenfalls wird das gebildete Konjugat anschließend gereinigt, z.B. durch Ultrazentrifugation oder Gelfiltration.

Die nach dem Stand der Technik eingesetzten Stabilisatoren binden adsorptiv an die freien Oberflächen der Metallpartikel. Durch längere Lagerung oder durch Änderung der Umgebungsbedingungen, wie sie z.B. in einem Test durch Kontakt mit Probe (Blut, Serum, Plasma, Urin), Einbringen der Konjugate in Teststreifenvliese etc. auftreten, können die Stabilisatoren mehr oder weniger stark von den Oberflächen desorbieren oder verdrängt werden. Dies führt zu einer Verschlechterung der Aggregationsstabilität und zu einer Zunahme der unspezifischen Reaktivität. Darüber hinaus handelt es sich bei einem großen Teil der verwendeten Stabilisatoren um schlecht definierte Produkte mit zum Teil schwankender Qualität, z.B. Rinderserumalbumin, Gelatine. Dadurch können auch Schwankungen in der Stabilisierungswirkung auftreten.

Adsorptionsvorgänge an Partikeloberflächen sind sehr komplex und bisher nur zum Teil verstanden. Es wird angenommen, dass die Adsorption über eine Kombination von elektrostatischen Wechselwirkungen, Van-der-Waals-Kräften und hydrophoben Wechselwirkungen zustande kommt (Beesley, Supra). Je nach Art des adsorbierten Biomoleküls kann dabei die eine oder die andere Bindungsart überwiegen.

Bei der Protein-Gold-Konjugation nach bekannten Techniken entstehen in einem gewissen Ausmaß Aggregate. Diese unerwünschten Aggregate treten häufig schon vor der Zugabe des Stabilisierungsmittels auf. Die Ursachen für die Bildung der Aggregate können z.B. darin liegen, dass "klebrige" Proteine, d.h. Proteine mit einer hydrophoben Oberfläche, aneinander binden und dadurch auch die Goldpartikel, mit denen sie konjugiert sind, verbrücken. Es ist deshalb z.B. beschrieben, IgG-Präparationen vor der Kopplung an Gold durch Ultrazentrifugation von Aggregaten zu befreien (W.D. Geoghegan, G.A. Ackerman, J. Histochem. Cytochem. 25 (1977), 1187-1200). Weiterhin ist es möglich, dass nicht durch Protein abgedeckte, hydrophobe Stellen (hydrophobic patches) auf der Oberfläche von Edelmetallpartikeln und insbesondere Goldpartikeln miteinander in Wechselwirkung treten und Partikel-Aggregate bilden. Eine weitere Ursache für das Zustandekommen der unerwünschten Aggregate kann auch darin liegen, dass nicht durch Protein abgedeckte, hydrophobe Stellen auf der Edelmetalloberfläche mit hydrophoben Stellen auf den an benachbarte Goldpartikel konjugierten Proteinen wechselwirken und so Goldpartikel miteinander vernetzen.

Häufig beobachtet man auch ein Nachvernetzen von Protein-Gold-Konjugaten, welches auch auftritt, wenn die Konjugate bereits mit einem Stabilisierungsmittel abgesättigt worden sind. Dies ist wahrscheinlich darauf zurückzuführen, dass auch nach Einwirkung der herkömmlichen Stabilisierungsmittel hydrophobe Stellen auf der Goldoberfläche oder/und auf dem konjugierten Protein verbleiben, über die mit langsamer Kinetik eine unkontrollierte Aggregation von Protein-Gold-Partikeln erfolgt. Diese Probleme treten nicht nur bei Konjugaten mit Partikeln aus Gold auf, sondern auch bei Partikeln aus anderen Feststoffen, insbesondere anderen Metallen.

Aufgabe der vorliegenden Erfindung war es daher, Konjugate aus kolloidalen Partikeln und Biomolekülen in einer stabilen Form bereitzustellen, welche die Nachteile des Standes der Technik nicht aufweist.

Überraschenderweise wurde nun gefunden, dass sich Detergenzien hervorragend zur Stabilisierung von Biomolekül-Partikel-Konjugaten eignen. Ein Gegenstand der Erfindung Ist deshalb ein Verfahren zur Herstellung einer Zusammensetzung, welche kolloidale Partikel, an deren Oberfläche Biomoleküle adsorbiert sind, und ein Detergenz umfasst, wobei man zu einer Biomoleküle enthaltenden Lösung vor der Beladung der kolloidalen Partikel mit dieser Lösung ein Detergenz zusetzt.

Es wurde gefunden, dass der Zusatz von Detergenzien zu kolloidalen Partikeln, zum Beispiel zu einer Biomoleküle enthaltenden Lösung vor der Beladung der kolloidalen Partikel mit dieser Lösung die Bindung an die Partikel deutlich weniger beeinträchtigt als der Fachmann erwartet hätte, sondern überraschenderweise in mehrfacher Hinsicht vorteilhaft ist. Durch die erfindungsgemäße Zugabe von Detergenz werden bereits vor der Konjugation und vor der Nachbeladung mit herkömmlichem Stabilisierungsmittel stattfindende Aggregationsprozesse verhindert, was zu einer besseren Reproduzierbarkeit des Herstellungsverfahrens der Konjugate und zu einer einheitlicheren Größenverteilung der Konjugate, d.h. einer erhöhten Monodispersität, führt.

Im Vergleich zu den Stabilisatoren des Standes der Technik wird eine wesentliche Verbesserung hinsichtlich der Stabilität der Konjugate erreicht. Insbesondere wird die langsame Nachaggregation bereits stabilisierter Biomolekül-Partikel-Konjugate, wie sie bei herkömmlichen Zusammensetzungen auftritt, unterdrückt. Dies führt zu einer verbesserten Langzeitstabilität und einer geringeren Aggregationsneigung in Lösung, zu einer besseren Stabilität gegenüber Veränderungen der Umgebungsbedingungen und zu einer verbesserten Testfunktion, z.B. verbesserten Chromatographieeigenschaften. Durch die Zugabe von Detergenz wird die Funktion der Biomolekül-Partikel-Konjugate und insbesondere die Funktion der Biomolekül-Gold-Konjugate in Tests deutlich verbessert. So wird beispielsweise die nicht spezifische Bindung, was dem Leerwert im Test entspricht, verringert.

Besonders überraschend war, dass die Verbesserungen erreicht werden konnten, ohne dass dabei die Funktion der Konjugate, beispielsweise durch Verdrängung der Biomoleküle durch das Detergenz oder durch mögliche Wechselwirkungen der Biomoleküle oder der kolloidalen Partikel mit dem Detergenz negativ beeinflusst wurde.

Die durch das erfindungsgemäße Verfahren erhältliche Zusammensetzung kann als wässrige Suspension oder auch immobilisiert, beispielsweise auf einem Chromatografiematerial, wie etwa einem saugfähigen Papier, vorliegen.

Die Partikel können metallische oder nichtmetallische Partikel wie etwa Carbonpartikel sein. Bevorzugt sind metallische Partikel wie etwa Partikel aus Metallen, Metalloxiden, Metallhydroxiden, Metallverbindungen, oder mit Metallen oder Metallverbindungen beschichtete Partikel. Besonders bevorzugt sind Metallpartikel.

Die Metallpartikel sind vorzugsweise Edelmetallpartikel, z.B. Metalle, ausgewählt aus der Gruppe, bestehend aus Gold, Silber, Kupfer, Platin, Palladium und Mischungen davon. Besonders bevorzugt sind Goldpartikel.

Der mittlere Durchmesser der Partikel liegt - wie im Stand der Technik üblich - im Bereich von 1 bis 1000 nm und kann je nach Anwendungszweck variiert werden. Bevorzugt ist der mittlere Durchmesser der Partikel im Bereich von 2 bis 200 nm und besonders bevorzugt von 2 bis 100 nm.

Die an der Oberfläche der Partikel adsorbierten Biomoleküle sind vorzugsweise ausgewählt aus der Gruppe, bestehend aus Proteinen, Glycoproteinen, Peptiden, Nucleinsäuren, peptidischen Nucleinsäuren, Sacchariden, Antigenen und Haptenen. Besonders bevorzugt werden Biomoleküle, ausgewählt aus der Gruppe, bestehend aus Antikörpern, Antikörperfragmenten, Lectinen, Enzymen, Streptavidin, Avidin, Protein A, Antigenen, wie etwa rekombinanten Polypeptiden oder multiplen Antigenen (vgl. WO96/03652), z.B. Polyhaptenen (mehrere an einen Träger wie etwa Dextran oder ein Protein gekoppelte Haptene oder Peptide), Peptiden und Haptenen (niedermolekulare Substanzen, vorzugsweise mit einem MW ≤ 1500 wie etwa Biotin, Fluorescein oder Digoxigenin). Bezüglich der genauen Bedingungen zur Adsorption dieser Biomoleküle an Goldpartikel wird auf die bereits genannten Übersichtsartikel De Mey und Beesley verwiesen.

Erfindungsgemäß können als Detergenzien anionische, kationische, ampholytische oder nicht ionische Grenzflächen-aktive Substanzen, insbesondere Tenside, eingesetzt werden. Bevorzugt umfassen die erfindungsgemäßen Zusammensetzungen als Detergenz ein Ethoxylatdetergenz und besonders bevorzugt Polyethoxysorbitanlaurat oder/und -oleat oder/und Laurylpolyethylenglykolether. Diese Detergenzien sind unter den Markennamen TWEEN® sowie Brij® (z.B. TWEEN 80, TWEEN 20, Brij 35) kommerziell erhältlich.

Bevorzugt wird das Detergenz in einer Konzentration eingesetzt, bei der die kritische Micellkonzentration nicht überschritten ist. Unter der kritischen Micellkonzentration wird dabei diejenige Konzentration verstanden, bei der sich aus den Tensidmolekülen übergeordnete Aggregate, sogenannte Micellen bilden. Das Erreichen der kritischen Micellkonzentration kann leicht durch die sprunghafte Änderung physikalischer Eigenschaften, wie beispielsweise der Oberflächenspannung, des osmotischen Drucks, der Äquivalentleitfähigkeit, der Grenzflächenspannung und/oder der Dichte bestimmt werden. Diese Parameter können jeweils mit bekannten Methoden gemessen werden.

Die optimale Detergenzkonzentration hängt jeweils von den Eigenschaften des zu konjugierenden Biomoleküls ab und muss für jedes Biomolekül individuell ermittelt werden. Die optimale Detergenzkonzentation liegt vor, wenn einerseits eine ausreichende Menge an Biomolekülen an die Oberfläche der kolloidalen Partikel bindet und andererseits unspezifische hydrophobe Wechselwirkungen weitgehend unterdrückt werden. Bevorzugt liegt das Detergenz in einer Konzentration von 0,0001 bis 1 mM, besonders bevorzugt von 0,001 bis 0,1 mM vor (Endkonzentration im Konjugationsansatz).

Die durch das erfindungsgemäße Verfahren erhältliche Zusammensetzung kann durch die Zugabe eines Detergenz zu einer Biomoleküle enthaltenden Lösung vor der Beladung der kolloidalen Partikel mit dieser Lösung hergestellt werden.

Die durch das erfindungsgemäße Verfahren erhältlichen Zusammensetzungen können als Nachweisreagenz, insbesondere als immunologisches Nachweisreagenz, verwendet werden. In einer ersten bevorzugten Ausführungsform wird das Nachweisreagenz in einem Immunoassay eingesetzt, d.h. in einem Verfahren zur Bestimmung eines Analyten in einer Probeflüssigkeit mittels immunologischer Methoden, z.B. durch einen kompetitiven Assay, bei dem ein markiertes Analytenanalog oder ein markierter, Analyt-spezifischer Rezeptor', z.B. ein Antikörper eingesetzt wird, oder einen Sandwich-Assay, bei dem ein markierter Analyt-spezifischer Rezeptor oder ein markierter, mit dem Analyt-spezifischen Rezeptor bindefähiger weiterer Rezeptor eingesetzt wird. Bevorzugte Beispiele sind Schwangerschaftstests, z.B. Tests zum Nachweis von Humanchoriongonadotropin (HCG), oder Verfahren zum Nachweis von Drogen wie etwa Kokain oder Amphetaminen, Humanserumalbumin, Troponin T, Myoglobin und Immunglobulinen wie etwa Anti-HIV-Antikörpern. Besonders bevorzugte Anwendungsformen sind Schnelltests, bei denen die zu bestimmende Probe auf ein saugfähiges, das Nachweisreagenz enthaltende Material, z.B. einen Teststreifen, gegeben wird. Eine zweite besonders bevorzugte Ausführungsform, bei der die erfindungsgemäß stabilisierte Zusammensetzung verwendet werden kann, ist die Anfärbung von Gewebeschnitten.

Außerdem können die durch das erfindungsgemäße Verfahren erhältlichen Zusammensetzungen natürlich auch für alle weiteren Anwendungen eingesetzt werden, die für Biomolekül-Partikel-Konjugate bekannt sind, z.B. zum Gentransfer.

Gegenstand der vorliegenden Erfindung ist daher ein Verfahren zur Stabilisierung von Konjugaten aus kolloidalen Partikeln und Biomolekülen, wobei man zu einer Biomoleküle enthaltenden Lösung vor der Beladung der kolloidalen Partikel, insbesondere Goldsol, mit dieser Lösung ein Detergenz zugibt. Auf diese Weise kann eine erhöhte Langzeitstabilität der Konjugate sowie eine verbesserte pH-Stabilität und eine verbesserte Stabilität gegenüber der Anwesenheit anderer Substanzen erreicht werden. Bevorzugt wird dabei das Detergenz in einer Menge zugesetzt, bei der die kritische Micellkonzentration nicht überschritten wird. Vorzugsweise wird das Detergenz in einer Menge zugesetzt, sodass sich eine Endkonzentration von 0,0001 bis 1 mM, bevorzugt 0,001 bis 0,1 mM ergibt.

Weiterhin kann man nach der Beladung zusätzliche, aus dem Stand der Technik bekannte Stabilisatoren, wie etwa inerte Proteine, z. B. Rinderalbumin, oder/und Polyethylenglykole einsetzen.

Die Erfindung wird durch die nachfolgenden Beispiele weiter erläutert.

### Beispiel 1 (Vergleichsbeispiel)

### Herstellung von Protein-Goldkonjugaten

Eine Lösung des an Goldpartikel zu adsorbierenden Proteins wurde gegen einen geeigneten Beladepuffer dialysiert oder mit dem Beladepuffer verdünnt. Anschließend wurden eventuell gebildete Aggregate durch Zentrifugation oder Filtration durch einen 0,2 µm Filter entfernt.

Der pH-Wert der die kolloidalen Goldpartikel enthaltenden Lösung wurde mit K₂CO₃ auf den pH-Wert der Proteinlösung eingestellt. Dann wurde die Proteinlösung unter Rühren der kolloidalen Goldlösung zugesetzt. Das Volumenverhältnis Proteinlösung zu kolloidaler Goldlösung war dabei 1 : 10.

Die Stabilisierung der auf diese Weise hergestellten Protein-Gold-Konjugate gemäß dem Stand der Technik erfolgte durch Zugabe einer RSA-Lösung bis zu einer Endkonzentration von 0,01 % bis 3 % (w/v) oder durch Zugabe einer Polyethylenglykollösung bis zu einer Endkonzentration von 0,01 % bis 0,1 % (w/v).

Anschließend erfolgte eine Reinigung und Aufkonzentrierung der Protein-Gold-Konjugate sowie die Einstellung der gewünschten Lagerpufferbedingungen, z.B. 20 mM Tris, 100 mM NaCl pH 8, 1 % RSA oder/und 0,01 % bis 0,1 % PEG, NaN₃.

### Beispiel 2

Einfluss des Zeitpunkts der Detergenzzugabe während der Konjugation von PAK < Digoxin > S-IgG (IS) (Erklärung: polyklonaler Anti-Digoxin-Antikörper vom Schaf, immunosorbierte IgG-Präparation) an 20 nm Goldsol.

Der hydrophobe Antikörper PAK < Digoxin > S-IgG (IS) wurde an ein Goldsol mit einem Partikeldurchmesser von 20 nm konjugiert. Die Konjugation fand in einer Lösung mit einem pH-Wert von 8,5 statt. Ausgehend von einem als M843 bezeichneten Goldsol wurde unter Zugabe des hydrophoben Antikörpers in einer Endkonzentration von 5 µg/ml ein Protein/Gold-Konjugat gebildet. Der Versuchsansatz 2a stellt dabei ein gemäß dem Stand der Technik ohne Detergenzzugabe hergestelltes Konjugat dar. Zu den Versuchsansätzen 2b bis 2e wurde zu unterschiedlichen Zeitpunkten 0,04 mM Brij 35 zugesetzt. In der Tabelle 1 ist jeweils der Quotient aus OD bei 550 nm und bei 600 nm gegeben. Dieser Wert ist eine Kenngröße für die Einheitlichkeit der Größenverteilungen. Je niedriger dieser Wert ist, desto höher ist der Anteil an Aggregaten und desto uneinheitlicher ist die Größenverteilung der Konjugatpartikel. Entsprechend ist ein möglichst hoher Wert von OD 550/600 erwünscht. Weiterhin ist in Tabelle 1 der mittlere Durchmesser der Konjugatpartikel, gemessen mit Photonenkorrelations-Spektroskopie unter der Bezeichnung PCS angegeben. Der Durchmesser der Konjugatpartikel und somit der Wert PCS soll möglichst gering sein.

Die Verfahrensführung war im Allgemeinen so, dass zunächst das unbeladene Ausgangsgoldsol mit dem IgG zur Bildung des Biomolekül-Gold-Konjugats versetzt wurde. Nach Zugabe der IgG-Lösung zum Goldsol wurde das Konjugat mit RSA (Rinderserumalbumin) als Stabilisierungsmittel nachbeladen. Beim Versuchsansatz 2b wurde das Detergenz vor Zugabe des IgG zur Goldlösung zugesetzt (Die IgG-Lösung wurde bei Zugabe zum Goldsol 1:10 verdünnt); in Versuchsansatz 2c wurde das Detergenz der IgG-Lösung zugesetzt; in Versuchsansatz 2d wurde das Detergenz unmittelbar nach Zugabe der IgG-Lösung zum Goldsol, aber vor der Nachbeladung mit RSA zugesetzt und in Versuchsansatz 2e wurde das Detergenz nach abgeschlossener Nachbehandlung mit RSA zugesetzt.

**Tabelle 1**

| PAK < Digoxin > S-IgG (IS; 07)-Gold-Konjugate | | | | |
|---|---|---|---|---|
| Versuchsansatz | IgG-Konz. [µg/ml] | OD 550/600 | PCS [nm] | Bemerkungen |
| M843 | - | 3,94 | 21,5 ± 3,7 | GOLDSOL |
| 2a | 5 | 3,87 | 107 ± 42 | (Vergleichsbeispiel) |
| 2b | 5 | 4,08 | 65 ± 33 | = 2a, aber zusätzl. 0,04 mM Brij 35 vor IgG |
| 2c | 5 | 4,06 | 58 ± 30 | = 2a, aber zusätzl. 0,4 mM Brij 35 in IgG-Lsg |
| 2d | 5 | 3,9 | 100 ± 46 | = 2a, aber zusätzl. 0,04 mM Brij 35 nach IgG |
| 2e | 5 | 3,8 | 94 ± 46 | = 2a, aber zusätzl. 0,04 mM Brij 35 nach RSA |

Wie der Tabelle 1 zu entnehmen ist, weisen die Versuchsansätze 2b und 2c die günstigsten Eigenschaften auf. Die erhaltenen Konjugate weisen einen gegenüber dem Stand der Technik deutlich verbesserten Durchmesser und einen hohen OD 550/600 Wert, d.h. eine relativ einheitliche Größenverteilung der Partikel auf. Durch die Zugabe eines Detergenz vor oder/und während der Beladung des Goldsols werden somit die auf hydrophoben Kräften beruhenden PAK-PAK-, Gold-Gold- und unspezifischen PAK-Gold-Wechselwirkungen am besten unterdrückt.

### Beispiel 3

### Einfluss des Zeitpunkts der Detergenzzugabe bei der Konjugation von rekombinantem HIV-Antigen p24 an 40 nm Goldsol

Goldsol mit einer durchschnittlichen Partikelgröße von 40 nm (M 825) wurde mit dem rekombinanten HIV-Antigen p24 in einer Konzentration von 5 µg/ml beladen. Der pH-Wert der Lösungen wurde jeweils auf 8,0 eingestellt. Die Verfahrensführung war ähnlich der in Beispiel 2 beschriebenen. Durch Zugabe des Antigens p24 zu dem Goldsol wurden p24-Gold-Konjugate gebildet. Anschließend wurde durch Zugabe von RSA als Stabilisierungsmittel eine Nachbeladung durchgeführt. In Versuchsansatz 3b wurde zusätzlich 0,04 mM Brij 35 zum Goldsol vor der p24-Zugabe zugesetzt; in Versuchsansatz 3c wurde 0,4 mM Brij 35 zur p24-Lösung zugesetzt (die p24-Lösung wurde bei der anschließenden Zugabe zum Goldsol 1 : 10 verdünnt) und in Versuchsansatz 3d wurde 0,04 mM Brij 35 nach abgeschlossener Nachbeladung mit RSA zugesetzt.

**Tabelle 2**

| p24(02)-Gold-Konjugate | | | | |
|---|---|---|---|---|
| Versuchsansatz | Bemerkungen | Ausbeute [%] | OD 550/600 | PCS [nm] |
| M 825 | Goldsol | 100 | 2,41 | 43 ± 24 |
| 3a | (Vergleichsbeispiel) | 78 | 2,06 | 83 ± 45 |
| 3b | = 3a, aber zusätzl. 0,04 mM Brij 35 vor p24-Zugabe | 93 | 2,12 | 63 ± 36 |
| 3c | = 3a, aber zusätzl. 0,4 mM Brij 35 in p24-Lösung | 92 | 2,13 | 63 ± 35 |
| 3d | = 3a, aber zusätzl. 0,04 mM Brij 35 nach RSA-Zugabe | 85 | 2,01 | 67 ± 24 |

Auch hier ist eine deutliche Verbesserung der Parameter OD 550/600 sowie PCS im Vergleich zu Versuchsansatz 3a ohne die Zugabe von Brij 35 zu sehen.

### Beispiel 4

### Zugabe von unterschiedlichen Detergenzkonzentrationen bei der Konjugation von MAK < PSA > M-10-IgG (muriner monoklonaler Anti-PSA-Antikörper Nr. 10, IgG-Präparation) an 20 nm Goldsol

Es wurden MAK < PSA > M-10-IgG-Goldkonjugate durch Zugabe von MAK < PSA > M-10-IgG an 20 nm Goldsol gebildet. Das Detergenz wurde in den angegebenen Konzentrationen der MAK < PSA > M-10-IgG-Lösung zugesetzt. Die Ergebnisse sind in Tabelle 3 dargestellt.

Für den MAK < PSA > M-10-IgG wurde eine finale Brij-Konzentration von 0,005 bis 0,01 mM als optimal ermittelt. Insgesamt wurde festgestellt, dass bei hydrophoberen Proteinen eine höhere Detergenzkonzentration benötigt wird als bei weniger hydrophoben.

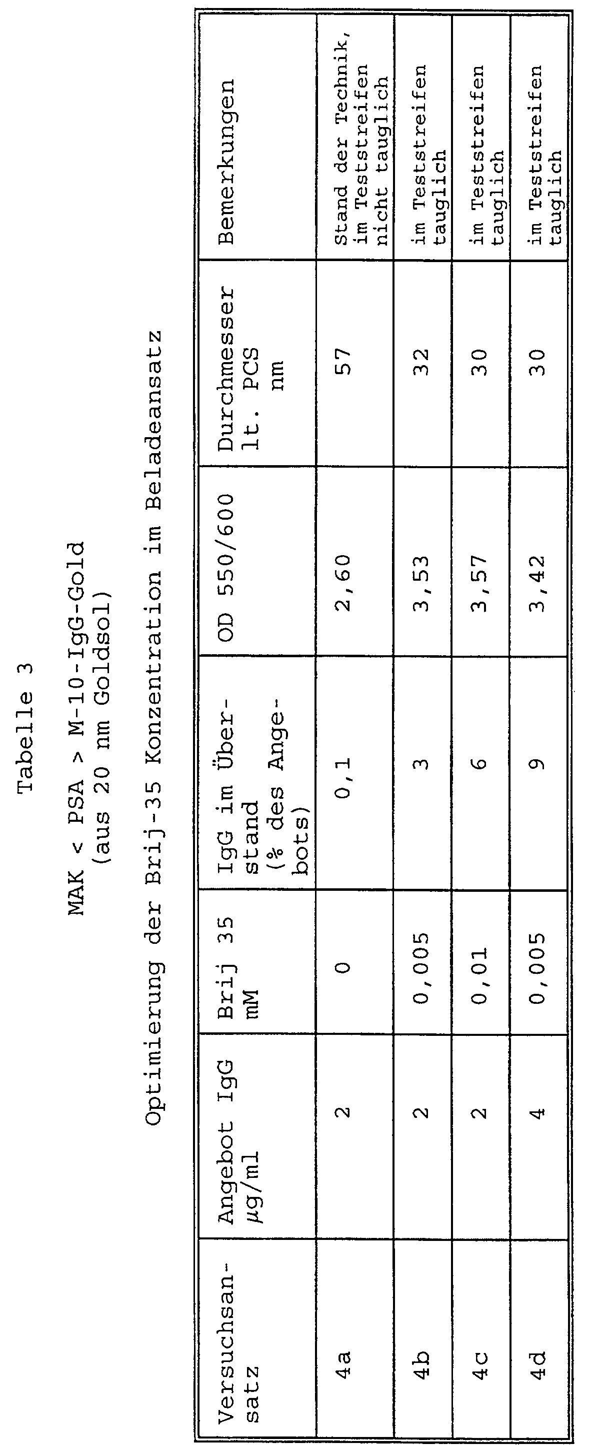

### Beispiel 5

### Stabilität der Konjugate

Es wurde die Stabilität erfindungsgemäß hergestellter Goldkonjugate im Vergleich zu konventionell hergestellten Goldkonjugaten untersucht. Ausgehend von jeweils dem gleichen MAK < HCG >-IgG und 40 nm Goldsol wurde in zwei Ansätzen nach dem gleichen Verfahren ein MAK < HCG > IgG-Gold-Konjugat hergestellt. Bei Ansatz 5A erfolgte die Herstellung durch Zugeben der Proteinlösung zum Goldsol ohne den Zusatz von Detergenz, bei Ansatz B enthielt die zum Beladen des Goldsols verwendete IgG-Lösung 0,1 mM Brij 35. Die kennzeichnenden Parameter OD 550/660 und der Durchmesser PCS wurden jeweils unmittelbar nach Fertigstellung des Konjugats, d.h. nach Zusatz von RSA als Stabilisierungsmittel als auch nach Lagerung bei 4 °C in regelmäßigen Zeitintervallen bis zu 27 Wochen untersucht. Bei Ansatz 5A nahm die Partikelgröße im Verlauf der Lagerung deutlich zu und der Wert für OD 550/600 ab, während beim erfindungsgemäßen Ansatz 5B sich beide Parameter nur geringfügig veränderten. Dies zeigt, dass die erfindungsgemäßen Protein-Gold-Konjugate wesentlich stabiler als die nach dem Stand der Technik hergestellten Protein-Gold-Konjugate sind.

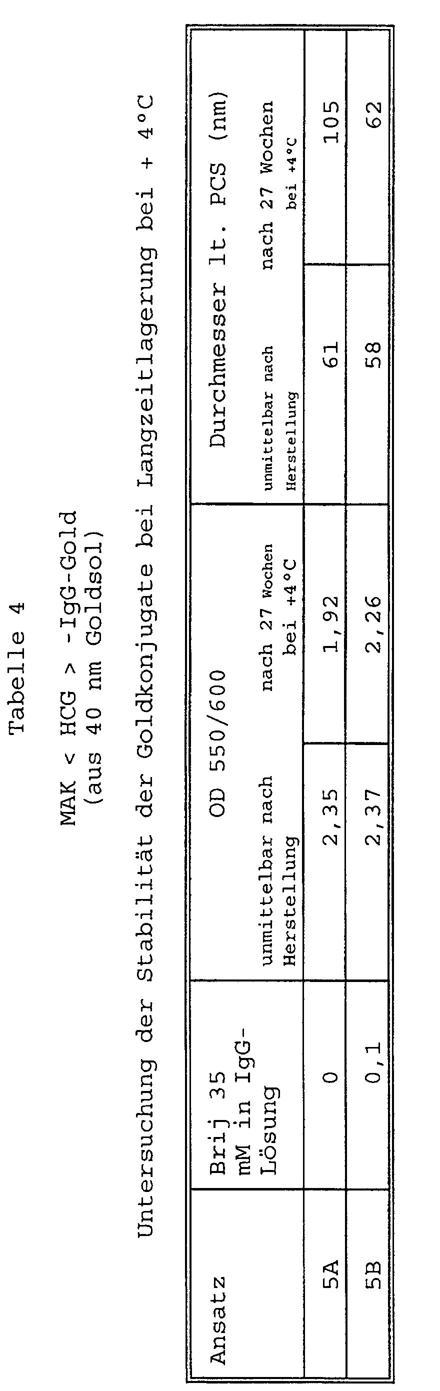

### Beispiel 6

### Einsatz von Goldkonjugaten in Troponin-Teststreifen

Eine Reihe von Konjugaten aus MAK < Troponin T > M-11-7-IgG und 40 nm Goldsol wurde hinsichtlich ihrer Funktion im Trop T-Teststreifen verglichen. Die Herstellung der Konjugate in Ansatz 6a und den Ansätzen 6b bis 6g unterschied sich im wesentlichen dadurch, dass bei Ansatz 6a ohne Detergenz, bei den Ansätzen 6b bis 6g jedoch mit 0,075 mM Brij 35 in der IgG-Lösung beladen wurde.

Wie aus der letzten Spalte von Tabelle 5 ersichtlich ist, ergeben sich mit den herkömmlich hergestellten Goldkonjugaten im Teststreifen nicht spezifische Leerwerte (= Blank), die so hoch sind, dass das Konjugat hier nicht eingesetzt werden kann. Die erfindungsgemäß in Gegenwart von Brij beladenen Konjugate ergeben hingegen keinen oder nur einen geringen Leerwert und sind somit für den Teststreifen tauglich.

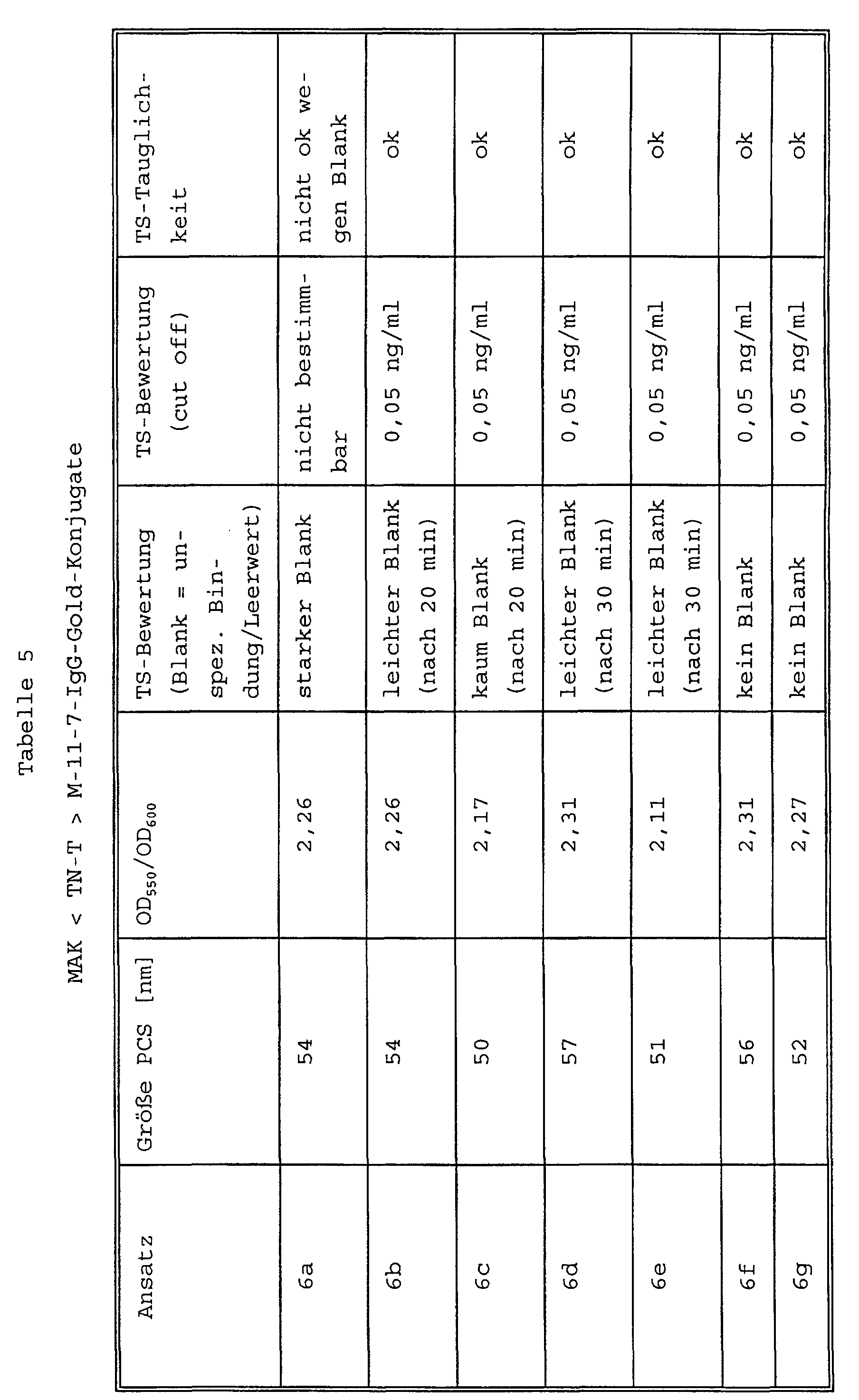

## Patentansprüche

1. Verfahren zur Herstellung einer Zusammensetzung, umfassend kolloidale Partikel, an deren Oberfläche Biomoleküle adsorbiert sind, und ein Detergenz,
**dadurch gekennzeichnet,**
**dass** man zu einer Biomoleküle enthaltenden Lösung vor der Beladung der kolloidalen Partikel mit dieser Lösung ein Detergenz zusetzt.

2. Verfahren nach Anspruch 1,
**dadurch gekennzeichnet,**
**dass** das Detergenz ein Ethoxylatdetergenz ist.

3. Verfahren nach Anspruch 1 oder 2,
**dadurch gekennzeichnet,**
**dass** das Detergenz in einer Konzentration von 0,0001 bis 1 mM eingesetzt wird.

4. Verfahren nach einem der vorhergehenden Ansprüche,
**dadurch gekennzeichnet,**
**dass** die Partikel Edelmetallpartikel sind.

5. Verfahren nach einem der vorhergehenden Ansprüche,
**dadurch gekennzeichnet,**
**dass** der mittlere Durchmesser der kolloidalen Partikel im Bereich von 1 nm bis 1000 nm liegt.

6. Verfahren nach einem der vorhergehenden Ansprüche,
**dadurch gekennzeichnet,**
**dass** die Biomoleküle ausgewählt sind aus der Gruppe, bestehend aus Proteinen, Glykoproteinen, Peptiden, Nukleinsäure, peptidischen Nukleinsäuren, Sacchariden, Antigenen und Haptenen.

7. Verfahren zur Stabilisierung von Konjugaten aus kolloidalen Partikeln und Biomolekülen,
**dadurch gekennzeichnet,**
**dass** man zu einer Biomoleküle enthaltenden Lösung vor der Beladung der kolloidalen Partikel mit dieser Lösung ein Detergenz zugibt.

8. Verfahren nach Anspruch 7,
**dadurch gekennzeichnet,**
**dass** man nach erfolgter Konjugation einen zusätzlichen Stabilisator zugibt.

## Claims

1. Process for the production of a composition comprising colloidal particles on the surface of which biomolecules are adsorbed, and a detergent
**wherein**
a detergent is added to a solution containing biomolecules before loading the colloidal particles with this solution.

2. Process as claimed in claim 1,
**wherein**
the detergent is an ethoxylate detergent.

3. Process as claimed in claim 1 or 2,
**wherein**
the detergent is used at a concentration of 0.0001 to 1 mM.

4. Process as claimed in one of the previous claims,
**wherein**
the particles are noble metal particles.

5. Process as claimed in one of the previous claims,
**wherein**
the mean diameter of the colloidal particles is in the range of 1 nm to 1000 nm.

6. Method as claimed in one of the previous claims,
**wherein**
the biomolecules are selected from the group comprising proteins, glycoproteins, peptides, nucleic acids, peptidic nucleic acids, saccharides, antigens and haptens.

7. Method for the stabilization of conjugates composed of colloidal particles and biomolecules,
**wherein**
a detergent is added to a solution containing biomolecules before loading the colloidal particles with this solution.

8. Method as claimed in claim 7,
**wherein**
an additional stabilizer is added after completion of the conjugation.

## Revendications

1. Procédé de préparation d'une composition comprenant des particules colloïdales sur la surface desquelles sont adsorbées des biomolécules et un détergent, **caractérisé en ce que** l'on ajoute un détergent à une solution contenant des biomolécules avant de charger les particules colloïdales avec cette solution.

2. Procédé selon la revendication 1, **caractérisé en ce que** le détergent est un détergent de type éthylate.

3. Procédé selon la revendication 1 ou 2, **caractérisé en ce que** l'on utilise le détergent à une concentration de 0,0001 à 1 mM.

4. Procédé selon l'une quelconque des revendications précédentes, **caractérisé en ce que** les particules sont des particules d'un métal précieux.

5. Procédé selon l'une quelconque des revendications précédentes, **caractérisé en ce que** le diamètre moyen des particules colloïdales est compris entre 1 nm et 1 000 nm.

6. Procédé selon l'une quelconque des revendications précédentes, **caractérisé en ce que** les biomolécules sont choisies dans le groupe constitué par des protéines, des glycoprotéines, des peptides, des acides nucléiques, des acides nucléiques peptidiques, des saccharides, des antigènes et des haptènes.

7. Procédé de stabilisation de conjugués de particules colloïdales et de biomolécules, **caractérisé en ce que** l'on ajoute un détergent à une solution contenant des biomolécules avant de charger les particules colloïdales avec cette solution.

8. Procédé selon la revendication 7, **caractérisé en ce que**, après avoir effectué la conjugaison, on ajoute un stabilisant supplémentaire.
